# EUROPEAN PATENT APPLICATION

(11) **EP 4 542 223 A1**
(43) Date of publication of application: **23.04.2025**
(21) Application number: 23219095.9
(22) Date of filing: 21.12.2023
(51) Int. Cl.: G01N 33/00, G01N 1/40

(54) **ODOR MEASUREMENT APPARATUS AND METHOD**

(30) Priority: 20.10.2023 KR 20230140803
(71) Applicant: HYUNDAI MOTOR COMPANY, Seoul 06797 (KR); Kia Corporation, Seocho-gu Seoul 06797 (KR)
(72) Inventor: LEE, Tae Hee, 18280 Hwaseong-si, Gyeonggi-do (KR); SUNG, Dae Un, 18280 Hwaseong-si, Gyeonggi-do (KR)
(74) Representative: Viering, Jentschura & Partner mbB Patent- und Rechtsanwälte

(57) **Abstract**

An odor measurement apparatus and an odor measurement method measure an odorous gas having a low concentration below a certain level and emitted from an explosive or a drug. The odor measurement apparatus and the odor measurement method concentrate the odorous gas having the low concentration below the certain level so as to make an odorous gas having a high concentration above a certain level, supply the odorous gas having the high concentration to an odor sensor, and enable the odor sensor to sense the odorous gas having the high concentration. The odor measurement apparatus and the odor measurement method are used in various industrial and inspection sites.

## Description

### TECHNICAL FIELD

The present disclosure relates to an odor measurement apparatus and an odor measurement method. More particularly, the present disclosure relates to an odor measurement apparatus and an odor measurement method capable of easily measuring a low concentration odorous gas.

### BACKGROUND

An existing odor sensor is configured to measure an odor generated not only in a vehicle but also in various industrial sites. The odor sensor may use an electrochemical odor sensor and an electrochemical odor sensor array. Alternatively, bio-odor sensors, such as a bio-peptide type sensor and a sensor using an amino acid, may be used.

Odor data measured by the existing odor sensor is acquired based on a predetermined time. Here, an odor component is not uniformly mixed in the air for a predetermined time, and the odor data measured by the odor sensor may vary depending on the temperature and humidity of air or gas containing an odor (referred to hereinafter as odorous gas). Accordingly, there is a problem in that measurement accuracy of the odor sensor may deteriorate.

Particularly, the existing odor sensor may detect an odorous gas having a concentration above a certain level. Meanwhile, there is a problem in that the existing odor sensor may not detect an odorous gas having a low concentration below a certain level when the odorous gas continuously and minutely leaks from various industrial sites, and the existing odor sensor may not detect another type of odorous gas having a low concentration below a certain level when the odorous gas is emitted from an explosive or a drug.

For example, since an explosive, a drug, and the like are sealed using various types of sealing means, the existing odor sensor may not detect an odorous gas having a low concentration below a certain level that is emitted from an explosive or a drug. In this case, a detection dog that detects an explosive and a drug is used.

In addition, an odorous gas having a low concentration below a certain level that minutely leaks from various industrial sites may be an explosive gas. In this case, gas accumulation may cause an explosion accident.

The above information disclosed in this Background section is provided only to enhance understanding of the background of the disclosure, and therefore it may contain information that does not form the prior art that is already known to a person of ordinary skill in the art.

### SUMMARY

The present disclosure provides an odor measurement apparatus and an odor measurement method configured to reliably measure an odorous gas having a low concentration below a certain level. In particular, the odorous gas, initially at a low concentration, is concentrated to achieve a higher concentration above a certain level, and then the concentrated odorous gas is supplied to an odor sensor, thereby enabling the odor sensor to precisely detect the odorous gas having low concentration.

In one aspect of the present disclosure, an odor measurement apparatus includes a prechamber having an inlet disposed at a first side of the prechamber and configured to allow an odorous gas to be introduced into the prechamber. The prechamber also has an outlet disposed at a second side of the prechamber and configured to allow the odorous gas to be discharged from the prechamber. The odor measurement apparatus also includes a flow pipe configured to connect the inlet to the outlet. The odor measurement apparatus also includes a gas trap mounted on the flow pipe and configured to collect odorous gas particles. The odor measurement apparatus also includes a first heater mounted on the prechamber and configured to heat the gas trap. The odor measurement apparatus also includes a first cooler mounted on the prechamber and configured to cool the gas trap. The odor measurement apparatus also includes an odor sensor chamber connected to the outlet of the prechamber through a connection pipe. The odor measurement apparatus also includes an odor sensor mounted in the odor sensor chamber and configured to sense the odorous gas desorbed from the gas trap. The odor measurement apparatus also includes a controller configured to perform operating control of the first heater and the first cooler.

In an embodiment, the odor measurement apparatus may further include a bypass pipe having a first end connected to the connection pipe and a second end connected to a discharge pipe of the odor sensor chamber. The odor measurement apparatus may further include a first three-way valve mounted at a connection point between the connection pipe and the first end of the bypass pipe and controlled by the controller. The odor measurement apparatus may further include a second three-way valve mounted at a connection point between the discharge pipe and the second end of the bypass pipe and controlled by the controller.

In another embodiment, the prechamber may have at least three inlets formed therein and configured to allow the odorous gas having a low concentration below a certain level to be smoothly introduced into the prechamber.

In still another embodiment, the discharge pipe of the odor sensor chamber may be connected to a suction pump driven under control of the controller.

In yet another embodiment, the odor sensor chamber may have a second heater and a second cooler mounted on the odor sensor chamber. The second heater may heat the odor sensor under control of the controller, and the second cooler may cool the odor sensor under the control of the controller.

In still yet another embodiment, the gas trap may be formed of a case and a porous body. The case is open at opposite sides of the case, and the porous body may be installed in the case so as to adsorb the odorous gas particles.

In a further embodiment, the gas trap may comprise a case, multilayer metal plates, and a plurality of metal pins. The case may be open at opposite sides of the case, the multilayer metal plates may be stacked in the case, and the plurality of metal pins may be mounted in a predetermined arrangement between the multilayer metal plates.

In another further embodiment, the gas trap may comprise a case and an electrostatic filter. The case may be open at opposite sides of the case, and the electrostatic filter may be installed in the case so as to adsorb the odorous gas particles.

In still another further embodiment, when the gas trap includes the electrostatic filter, the flow pipe may have a guidance device mounted on the flow pipe and configured to cause the odorous gas to reversely flow from a rear portion of the gas trap to a front portion of the gas trap.

In yet another further embodiment, the guidance device may include a first guidance pipe having a first end connected to a predetermined position of the flow pipe connected to the front portion of the gas trap. The first guidance pipe also has a second end connected to a predetermined position of the flow pipe connected to the rear portion of the gas trap. The guidance device may further include a second guidance pipe having a first end connected to the flow pipe between the first end of the first guidance pipe and the front portion of the gas trap. The second guidance pipe also has a second end connected to the flow pipe between the other end of the first guidance pipe and the outlet of the prechamber. The guidance device may further include a first opening/closing valve mounted on the flow pipe between the first end of the first guidance pipe and the first end of the second guidance pipe. The first opening/closing valve is opened or closed under control of the controller. The guidance device may further include a second opening/closing valve mounted on the flow pipe between the second end of the first guidance pipe and the second end of the second guidance pipe. The second opening/closing valve is opened or closed under control of the controller.

In another aspect of the present disclosure, an odor measurement method includes allowing an odorous gas having a low concentration below a certain level to flow through a flow pipe connecting an inlet of a prechamber to an outlet of the prechamber. The odor measurement method further includes collecting, by a gas trap mounted on the flow pipe, odorous gas particles for a predetermined time. The odor measurement method further includes desorbing the odorous gas particles collected in the gas trap after a predetermined time. The odor measurement method further includes supplying the odorous gas particles desorbed from the gas trap into an odor sensor chamber having an odor sensor installed therein. The odor measurement method further includes sensing, by the odor sensor, the odorous gas introduced into the odor sensor chamber.

In an embodiment, the prechamber may have a first cooler mounted on the prechamber. The odor measurement method further includes driving the first cooler under control of a controller so as to allow the odorous gas particles to be adsorbed on the gas trap through cooling of the gas trap when collecting, by the gas trap mounted on the flow pipe, the odorous gas particles for the predetermined time.

In another embodiment, the prechamber may have a first heater mounted on the prechamber. The odor measurement method further includes driving the first heater under control of a controller so as to allow the odorous gas particles to be desorbed from the gas trap through heating of the gas trap when desorbing the odorous gas particles collected in the gas trap after the predetermined time.

In still another embodiment, the odor sensor chamber may have a second cooler mounted on the odor sensor chamber. The odor measurement method further includes driving the second cooler under control of a controller so as to allow the odorous gas particles to be adsorbed on a surface of the odor sensor through cooling of the odor sensor when sensing, by the odor sensor, the odorous gas introduced into the odor sensor chamber.

In yet another embodiment, the odor sensor chamber may have a second heater mounted on the odor sensor chamber. The odor measurement method further includes driving the second heater under control of a controller so as to allow the odorous gas particles to be desorbed from a surface of the odor sensor through heating of the odor sensor after sensing, by the odor sensor, the odorous gas introduced into the odor sensor chamber.

In still yet another embodiment, collecting, by the gas trap mounted on the flow pipe, the odorous gas particles for the predetermined time may be repeatedly performed when a concentration of the odorous gas sensed by the odor sensor is below a certain level.

In a further embodiment, the odorous gas may be bypassed through a bypass pipe when the concentration of the odorous gas sensed by the odor sensor is below the certain level. The bypass pipe may connect a connection pipe connected to the outlet of the prechamber to a discharge pipe of the odor sensor chamber.

Other aspects and embodiments of the present disclosure are discussed below.

It is understood that the terms "vehicle", "vehicular", and other similar terms as used herein include motor vehicles in general, such as passenger automobiles including sport utility vehicles (SUV), buses, trucks, various commercial vehicles, watercraft including a variety of boats and ships, aircraft, and the like, and include hybrid vehicles, electric vehicles, plug-in hybrid electric vehicles, hydrogen-powered vehicles, and other alternative fuel vehicles (e.g. fuels derived from resources other than petroleum). As referred to herein, a hybrid vehicle is a vehicle that has two or more sources of power, for example, vehicles powered by both gasoline and electricity.

The above and other features of the present disclosure are discussed below.

### BRIEF DESCRIPTION OF THE DRAWINGS

In order that the disclosure may be well understood, there are now described various forms thereof, given by way of example, reference being made to the accompanying drawings, in which::
FIG. 1 and FIG. 2 are cross-sectional views showing an odor measurement apparatus according to an embodiment of the present disclosure and the operation state thereof;
FIG. 3A is a partially enlarged cross-sectional view showing a gas trap of the odor measurement apparatus according to the embodiment of the present disclosure;
FIG. 3B is a partially enlarged cross-sectional perspective view showing the gas trap of the odor measurement apparatus according to the embodiment of the present disclosure;
FIG. 4 is a flowchart showing an odor measurement method according to an embodiment of the present disclosure;
FIG. 5 and FIG. 6 are cross-sectional views showing an odor measurement apparatus according to another embodiment of the present disclosure and the operation state thereof; and
FIG. 7 is a flowchart showing an odor measurement method according to another embodiment of the present disclosure.

The drawings described herein are for illustration purposes only and are not intended to limit the scope of the present disclosure in any way.

It should be understood that the appended drawings are not necessarily to scale and present a somewhat simplified representation of various features illustrating the basic principles of the disclosure. The specific design features of the present disclosure as disclosed herein, including, for example, specific dimensions, orientations, locations, and shapes are determined in part by the particular intended application and use environment.

In the figures, same reference numbers refer to the same or equivalent parts of the present disclosure throughout the several figures of the drawing.

### DETAILED DESCRIPTION

Specific structural or functional descriptions given in connection with the embodiments of the present disclosure are merely illustrative for the purpose of describing embodiments according to the concept of the present disclosure. The embodiments according to the concept of the present disclosure may be implemented in various forms. Further, it should be understood that the present description is not intended to limit the present disclosure to the embodiments. On the contrary, the present disclosure is intended to cover not only the embodiments but also various alternatives, modifications, equivalents, and other embodiments, which may be included within the spirit and scope of the present disclosure as defined by the appended claims.

Meanwhile, in the present disclosure, terms such as "first" and/or "second" may be used to describe various components, but the components are not limited by the terms. The terms are used only for the purpose of distinguishing one component from other components. For example, a first component may be referred to as a second component, and similarly, a second component may also be referred to as a first component without departing from the scope of rights according to the concept of the present disclosure. When a component, device, element, or the like of the present disclosure is described as having a purpose or performing an operation, function, or the like, the component, device, element, or the like should be considered herein as being "configured to" meet that purpose or to perform that operation or function. Each of the component, device, element, and the like may separately embody or be included with a processor and a memory, such as a non-transitory computer readable media, as part of the apparatus.

Hereinafter, embodiments of the present disclosure are described in detail with reference to the accompanying drawings.

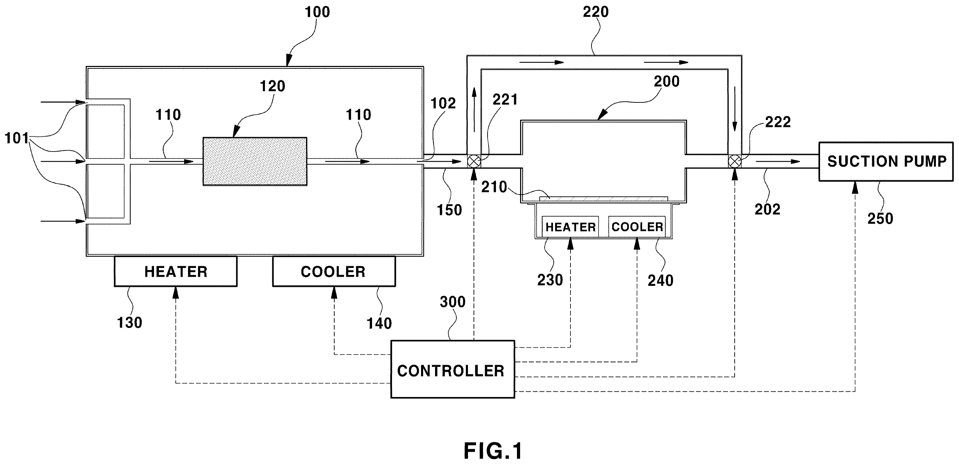

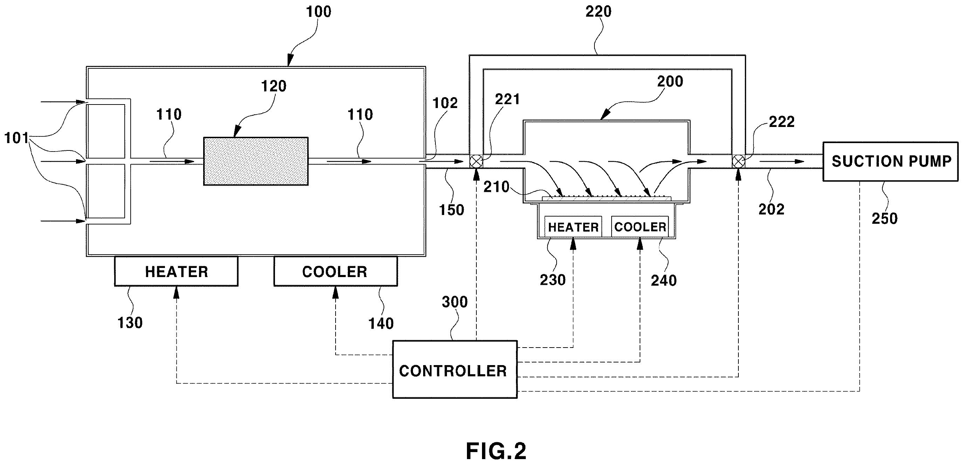

FIG. 1 and FIG. 2 are cross-sectional views showing an odor measurement apparatus according to an embodiment of the present disclosure and the operation state thereof.

As shown in FIG. 1 and FIG. 2, a prechamber 100 has an inlet 101 disposed at one side thereof and configured to allow an odorous gas to be introduced thereinto and has an outlet 102 disposed at the other side thereof and configured to allow the odorous gas to be discharged therefrom.

In addition, the prechamber 100 has a flow pipe 110 disposed therein and configured to connect the inlet 101 to the outlet 102 so as to allow the odorous gas to flow through the flow pipe 110.

In an embodiment, the prechamber 100 has at least three inlets 101 formed therein and configured to allow the odorous gas having a low concentration below a certain level to be smoothly introduced thereinto.

In this case, the front end of the flow pipe 110 may be formed of three pipes, each of which is communicably connected to a corresponding one of the inlets 101, and the rear end of the flow pipe 110 may be formed of one pipe communicably connected to three pipes.

Particularly, the flow pipe 110 has a gas trap 120 mounted therein and configured to collect odorous gas particles.

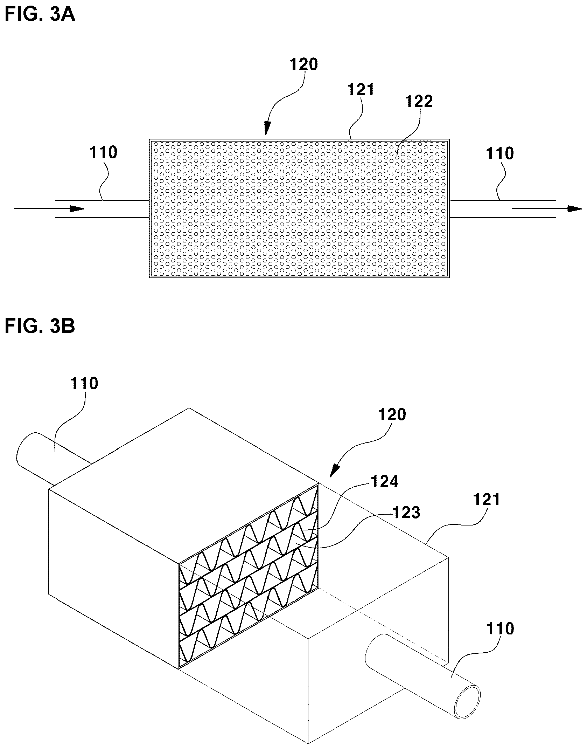

According to the embodiment of the present disclosure, as shown in FIG. 3A, the gas trap 120 comprises a case 121 open at the opposite sides thereof so as to be communicably connected to the flow pipe 110 and comprises a porous body 122 installed in the case 121 and configured to adsorb odorous gas particles, such as charcoal.

Alternatively, as shown in FIG. 3B, the gas trap 120 comprises the case 121 open at the opposite sides thereof so as to be communicably connected to the flow pipe 110, multilayer metal plates 123 stacked in the case 121, and a plurality of metal pins 124 mounted in a predetermined arrangement between the metal plates 123 and configured to adsorb odorous gas particles.

In addition, a first heater 130 adopted to heat the gas trap 120 and a first cooler 140 adopted to cool the gas trap are mounted at the lower portion of the prechamber 100.

For example, a heating coil or the like may be used as the first heater 130. As the first cooler 140, a blower or the like configured to suction cold air according to driving of an air conditioner may be used. Alternatively, a single Peltier element capable of performing heating or cooling may be adopted as the first heater 130 and the first cooler 140.

Meanwhile, at a high temperature above a certain degree, the frequency at which the odorous gas particles collide with and contact the surface of the porous body 122 or the metal pin 124 of the gas trap 120 decreases. As such, the speed at which the odorous gas particles are adsorbed on the surface of the porous body 122 or the metal pin 124 of the gas trap 120 decreases. The reason for this is that, as momentum of the odorous gas particles increases due to heat vibration, adsorption bonding force of the odorous gas particles with respect to the surface of the porous body 122 or the metal pin 124 of the gas trap 120 decreases.

Accordingly, at a high temperature above a certain degree, the odorous gas particles may not be easily adsorbed on the surface of the porous body 122 or the metal pin 124 of the gas trap 120, and the odorous gas particles may be easily desorbed from the surface of the porous body 122 or the metal pin 124 of the gas trap 120.

Conversely, at a low temperature below a certain degree, the frequency at which gas particles subjected to odor measurement collide with and contact the surface of the porous body 122 or the metal pin 124 of the gas trap 120 increases. As such, the speed at which the gas particles subjected to the odor measurement are adsorbed on the surface of the porous body 122 or the metal pin 124 of the gas trap 120 increases. The reason for this is that, as momentum of the odorous gas particles decreases due to heat vibration, adsorption bonding force of the odorous gas particles with respect to the surface of the porous body 122 or the metal pin 124 of the gas trap 120 increases.

Accordingly, at a low temperature below a certain degree, the odorous gas particles may be easily adsorbed on the surface of the porous body 122 or the metal pin 124 of the gas trap 120, and the odorous gas particles are not easily desorbed from the surface of the porous body 122 or the metal pin 124 of the gas trap 120.

Therefore, the gas trap 120 is cooled by the cooling operation of the first cooler 140 so as to cool the surface of the porous body 122 or the metal pin 124. Thus, the odorous gas particles may be easily adsorbed on the surface of the porous body 122 or the metal pin 124 of the gas trap 120.

Meanwhile, heat is transferred to the gas trap 120 by the heating operation of the first heater 130 so as to heat the surface of the porous body 122 or the metal pin 124. Thus, the odorous gas particles may be easily desorbed from the surface of the porous body 122 or the metal pin 124 of the gas trap 120.

An odor sensor chamber 200 is communicably connected to the outlet 102 of the prechamber 100 through a connection pipe 150 having a predetermined length.

In addition, an odor sensor 210 configured to sense an odorous gas is mounted in the odor sensor chamber 200.

Accordingly, the odor sensor 210 may easily sense the odorous gas desorbed from the gas trap 120 and then introduced into the odor sensor chamber 200 through the connection pipe 150.

In addition, the odor sensor chamber 200 may have a second heater 230 mounted thereon and configured to heat the odor sensor 210 and a second cooler 240 mounted thereon and configured to cool the odor sensor 210. Accordingly, as described above, the odorous gas particles may be easily adsorbed on the surface of the odor sensor 210 at a low temperature below a certain degree, and the odorous gas particles may be easily desorbed from the surface of the odor sensor 210 at a high temperature above a certain degree.

The odor measurement apparatus of the present disclosure further includes a bypass pipe 220 having one end connected to the connection pipe 150 and the other end connected to a discharge pipe 202 of the odor sensor chamber 200. The bypass pipe 220 further comprises a first three-way valve 221 mounted at a connection point between the connection pipe 150 and one end of the bypass pipe 220. The bypass pipe 220 further comprises a second three-way valve 222 mounted at a connection point between the discharge pipe 202 and the other end of the bypass pipe 220.

Accordingly, when the odorous gas particles are collected by the gas trap 120 for a predetermined time, the first three-way valve 221 may be opened in a direction of enabling the connection pipe 150 and the bypass pipe 220 to communicate with each other, and the second three-way valve 222 may be opened in a direction of enabling the bypass pipe 220 and the discharge pipe 202 to communicate with each other.

Alternatively, the first three-way valve 221 may be opened in a direction of enabling the connection pipe 150 and the odor sensor chamber 200 to communicate with each other so as to supply the odorous gas particles desorbed from the gas trap 120 into the odor sensor chamber 200, and the second three-way valve 222 may be opened in a direction of enabling the odor sensor chamber 200 and the discharge pipe 202 to communicate with each other.

Here, since a suction pump 250 driven under the control of a controller 300 is connected to the discharge pipe 202 of the odor sensor chamber 200, suction force resulting from driving of the suction pump 250 may be applied to the gas trap 120 in the prechamber 100 through the odor sensor chamber 200 and the connection pipe 150, and the suction force may be applied to the gas trap 120 in the prechamber 100 through the bypass pipe 220 and the connection pipe 150.

Meanwhile, the controller 200 is configured to perform ON or OFF control of the first heater 130 and the first cooler 140, perform ON or OFF control of the second heater 230 and the second cooler 240, and perform opening direction control for the first three-way valve 221 and the second three-way valve 222.

Hereinafter, a description is given as to an odor measurement method performed by the odor measurement apparatus according to the embodiment of the present disclosure.

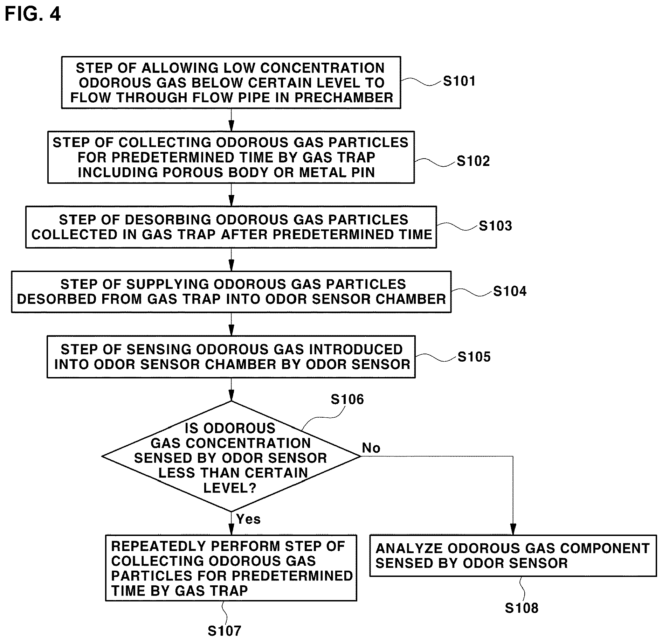

FIG. 4 is a flowchart showing the odor measurement method according to the embodiment of the present disclosure.

First, a low concentration odorous gas below a certain level is allowed to flow through the flow pipe 110 from the inlet 101 to the outlet 102 of the prechamber 100 for a predetermined time (S101).

In this case, since at least three inlets 101 of the prechamber 100 are provided, a low concentration odorous gas below a certain level may be smoothly introduced from an odor measurement target (for example, an explosive or a drug sealed with a sealing means) into the flow pipe 110 through three or more inlets 101.

Continuously, in the next step, odorous gas particles are collected for a predetermined time by the gas trap 120 mounted on the flow pipe 110 (S102).

In more detail, when the low concentration odorous gas introduced into the flow pipe 110 enters the case 121 of the gas trap 120, the odorous gas particles may be adsorbed and collected on the surface of the porous body 122 or the metal pin 124 in the case 121.

Further, when the odorous gas particles are easily collected for a predetermined time by the gas trap 120, the gas trap 120 is cooled by the cooling operation of the first cooler 140 under the control of the controller 300 so as to cool the surface of the porous body 122 or the metal pin 124. Thus, the odorous gas particles may be easily adsorbed on the surface of the porous body 122 or the metal pin 124 of the gas trap 120.

At this time, when the odorous gas particles are collected by the gas trap 120 for a predetermined time, the first three-way valve 221 may be opened under the control of the controller 300 in a direction of enabling the connection pipe 150 and the bypass pipe 220 to communicate with each other, and the second three-way valve 222 may be opened under the control of the controller 300 in a direction of enabling the bypass pipe 220 and the discharge pipe 202 to communicate with each other.

In addition, the suction pump 250 is driven under the control of the controller 300, and suction force resulting from the operation of the suction pump 250 is applied to the gas trap 120 in the prechamber 100 through the bypass pipe 220 and the connection pipe 150.

Accordingly, when the odorous gas particles are collected by the gas trap 120 for a predetermined time, the remaining odorous gas that is not collected does not flow to the odor sensor chamber 200 but flows to the discharge pipe 202 through the bypass pipe 220.

In the next step, the odorous gas particles collected in the gas trap 120 are desorbed after a predetermined time (S103).

To this end, the first heater 130 mounted on the prechamber 100 is controlled under the control of the controller 300 so as to desorb the odorous gas particles from the gas trap 120 through heating of the gas trap 120.

Accordingly, heat is transferred to the gas trap 120 by the heating operation of the first heater 130, and the surface of the porous body 122 or the metal pin 124 is heated. Thus, the odorous gas particles may be easily desorbed from the surface of the porous body 122 or the metal pin 124 of the gas trap 120.

In the next step, the odorous gas particles desorbed from the gas trap 120 are supplied into the odor sensor chamber 200 having the odor sensor 210 installed therein (S104).

To this end, the first three-way valve 221 may be opened under the control of the controller 300 in a direction of enabling the connection pipe 150 and the odor sensor chamber 200 to communicate with each other so as to supply the odorous gas particles desorbed from the gas trap 120 into the odor sensor chamber 200. The second three-way valve 222 may be opened under the control of the controller 300 in a direction of enabling the odor sensor chamber 200 and the discharge pipe 202 to communicate with each other,.

In addition, the suction pump 250 is driven under the control of the controller 300, and suction force resulting from the operation of the suction pump 250 is applied to the gas trap 120 in the prechamber 100 through the odor sensor chamber 200 and the connection pipe 150.

Accordingly, the odorous gas particles desorbed from the gas trap 120 may be supplied into the odor sensor chamber 200 without flowing to the bypass pipe 220 through the connection pipe 150.

In the next step, the odor sensor 210 senses the odorous gas introduced into the odor sensor chamber 200, i.e., the odorous gas desorbed from the gas trap 120 (S105).

To this end, the second cooler 240 mounted on the odor sensor chamber 200 is driven under the control of the controller 300 so that odorous gas particles are well adsorbed on the surface of the odor sensor 210 through cooling of the odor sensor 210.

Accordingly, as described above, the odor sensor 210 may be cooled by driving of the second cooler 240, and the odorous gas particles desorbed from the gas trap 120 may be easily adsorbed on the surface of the odor sensor 210 at a low temperature below a certain degree. Thus, the odor sensor 210 may easily sense the odorous gas desorbed from the gas trap 120.

After the step of sensing, by the odor sensor 210, the odorous gas introduced into the odor sensor chamber 200 for a predetermined time, it is desirable to desorb the odorous gas particles from the surface of the odor sensor 210 in order to sense the incoming odorous gas.

To this end, the second heater 230 mounted on the odor sensor chamber 200 is driven under the control of the controller 300 so that the odorous gas particles are well desorbed from the surface of the odor sensor 210 through heating of the odor sensor 210.

The second heater mounted on the odor sensor chamber is driven under the control of the controller so that the odorous gas particles are desorbed from the surface of the odor sensor through heating of the odor sensor 210.

Accordingly, as described above, the odor sensor 210 may be heated by driving of the second heater 230, and the odorous gas particles may be easily desorbed from the surface of the odor sensor 210 at a high temperature above a certain degree. Thus, the surface of the odor sensor 210 may be maintained in a clean state for sensing of the incoming odorous gas.

Meanwhile, in step S105 described above, a signal sensed by the odor sensor 210 is transmitted to the controller 300.

Next, the controller 300 determines whether the concentration of the odorous gas sensed by the odor sensor 210 is less than or equal to or greater than a certain level (S106).

As a result of the determination in step S106, when the concentration of the odorous gas sensed by the odor sensor 210 is less than the certain level (Yes in S106), the step of collecting, by the gas trap 120 mounted on the flow pipe 110, the odorous gas particles for a predetermined time is repeatedly performed (S107).

To this end, the first three-way valve 221 is opened again under the control of the controller 300 in the direction of enabling the connection pipe 150 and the bypass pipe 220 to communicate with each other. The second three-way valve 222 is opened again under the control of the controller 300 in the direction of enabling the bypass pipe 220 and the discharge pipe 202 to communicate with each other.

Therefore, when the odorous gas particles are repeatedly collected by the gas trap 120 for a predetermined time, the remaining odorous gas that is not collected does not flow to the odor sensor chamber 200, but the same is bypassed through the bypass pipe 220 so as to flow through the discharge pipe 202.

On the other hand, as a result of the determination in step S106, when the concentration of the odorous gas sensed by the odor sensor 210 is equal to or greater than the certain level (No in S106), an odorous gas component sensed by the odor sensor 210 may be analyzed (S108).

For example, the controller 300 may perform odorous gas analysis including analysis on a type of odorous gas component sensed by the odor sensor 210 and pattern comparison with a target gas, and the next step is performed depending on the analysis result.

Hereinafter, a description is given as to an odor measurement apparatus according to another embodiment of the present disclosure.

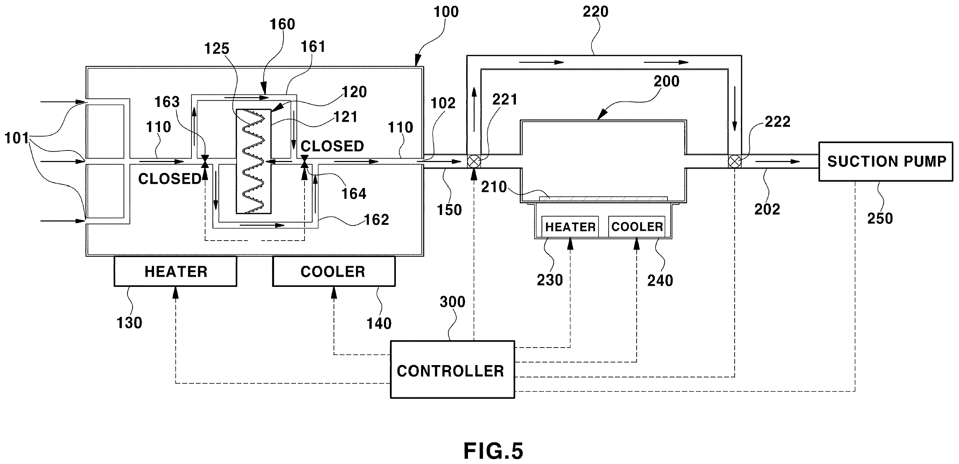

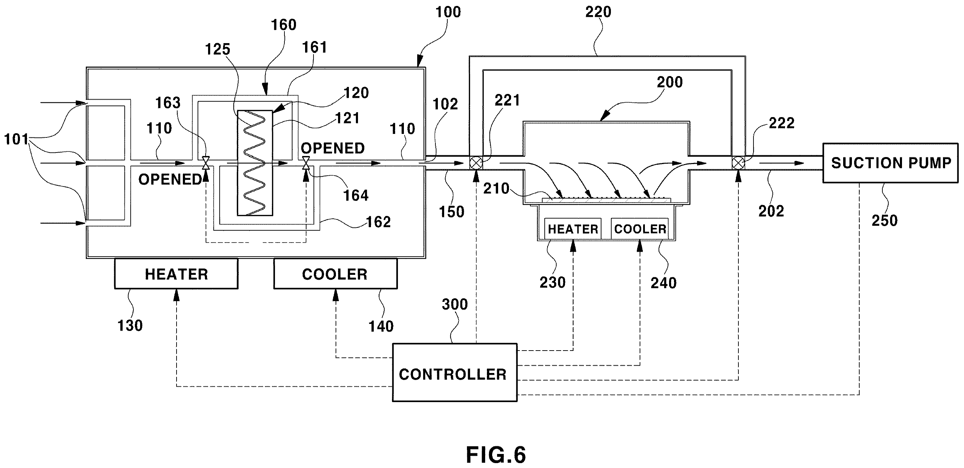

FIG. 5 and FIG. 6 are cross-sectional views showing the odor measurement apparatus according to another embodiment of the present disclosure and the operation state thereof.

The odor measurement apparatus according to another embodiment of the present disclosure is different from the odor measurement apparatus according to the embodiment only in that the gas trap 120 comprises an electrostatic filter, and the remaining components are the same as those of the odor measurement apparatus according to the embodiment.

According to another embodiment of the present disclosure, as shown in FIG. 5 and FIG. 6, the gas trap 120 comprises a case 121 open at the opposite sides thereof and an electrostatic filter 125 installed in the case 121 and configured to adsorb odorous gas particles.

For example, the electrostatic filter 125 may be a vehicle air conditioner filter.

At this time, after the odorous particles are adsorbed on the front portion of the electrostatic filter 125, even if the adsorbed odor particles are desorbed therefrom, the desorbed odor particles may not pass through the electrostatic filter 125. As a result, the desorbed odor particles do not flow to the odor sensor chamber 200.

Accordingly, when the gas trap 120 includes the electrostatic filter 125, the flow pipe 110 has a guidance device 160 mounted thereon and configured to cause the odorous gas to reversely flow from the rear portion of the filter 125 of the gas trap 120 to the front portion thereof.

As shown in FIG. 5 and FIG. 6, the guidance device 160 may include a first guidance pipe 161 and a second guidance pipe 162 connected to the flow pipe 110, and a first opening/closing valve 163 and a second opening/closing valve 164 mounted on the flow pipe 110.

The first guidance pipe 161 has one end connected to a predetermined position of the flow pipe 110 connected to the front portion of the gas trap 120 and the other end connected to a predetermined position of the flow pipe 110 connected to the rear portion of the gas trap 120.

The second guidance pipe 162 has one end connected to the flow pipe 110 between one end of the first guidance pipe 161 and the front portion of the gas trap 120 and has the other end connected to the flow pipe 110 between the other end of the first guidance pipe 161 and the outlet 102 of the prechamber 100.

The first opening/closing valve 163 is mounted on the flow pipe 110 between one end of the first guidance pipe 161 and one end of the second guidance pipe 162, and opening or closing of the first opening/closing valve 163 is controlled by the controller 300.

The second opening/closing valve 164 is mounted on the flow pipe 110 between the other end of the first guidance pipe 161 and the other end of the second guidance pipe 162, and opening or closing of the second opening/closing valve 164 is controlled by the controller 300.

Hereinafter, a description is given as to an odor measurement method performed by the odor measurement apparatus according to another embodiment of the present disclosure.

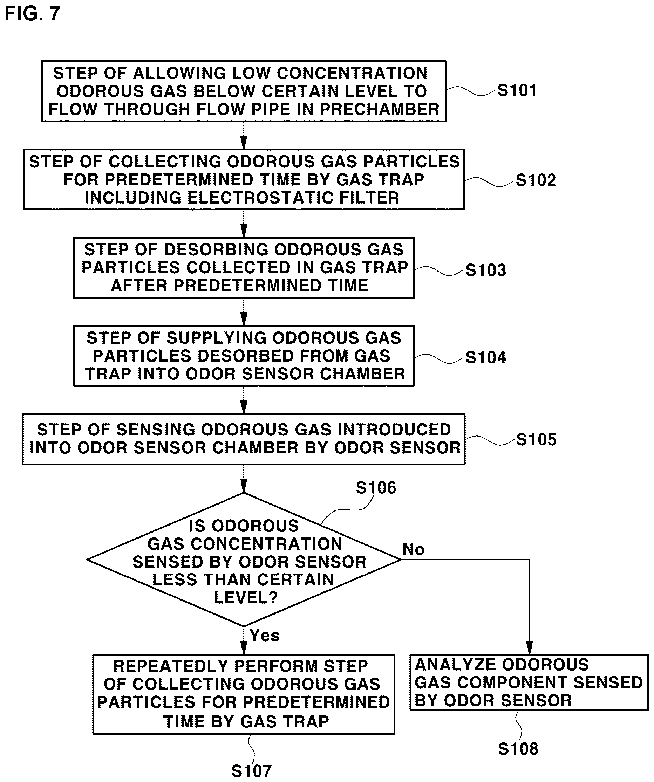

FIG. 7 is a flowchart showing the odor measurement method according to another embodiment of the present disclosure.

First, as described above, a low concentration odorous gas below a certain level is allowed to flow into the flow pipe 110 in the prechamber 100 (S101).

In the next step, odorous gas particles are collected for a predetermined time by the gas trap 120 including the electrostatic filter 125 (S102).

To this end, the first opening/closing valve 163 and the second opening/closing valve 164 are closed under the control of the controller.

In addition, the first three-way valve 221 may be opened under the control of the controller 300 in a direction of enabling the connection pipe 150 and the bypass pipe 220 to communicate with each other. The second three-way valve 222 may be opened under the control of the controller 300 in a direction of enabling the bypass pipe 220 and the discharge pipe 202 to communicate with each other.

Accordingly, after the low concentration odorous gas introduced into the flow pipe 110 passes through the first guidance pipe 161, the low concentration odorous gas reversely flows from the rear portion of the filter 125 of the gas trap 120 to the front portion thereof.

At this time, the gas trap 120 is cooled by the cooling operation of the first cooler 140 under the control of the controller 300, and thus the odorous gas particles may be easily adsorbed on the rear portion of the filter 125 of the gas trap 120.

Therefore, the odorous gas particles may be adsorbed on the rear portion of the electrostatic filter 125, and a remaining gas component that is not adsorbed passes through the filter 125 to flow through the second guidance pipe 162. Thereafter, the remaining gas component flows to the discharge pipe 202 along the bypass pipe 220.

In the next step, the odorous gas particles collected in the gas trap 120 including the electrostatic filter 125 are desorbed after a predetermined time (S103).

To this end, the first heater 130 mounted on the prechamber 100 is driven under the control of the controller 300 so as to desorb the odorous gas particles from the filter 125 of the gas trap 120 through heating of the gas trap 120.

Subsequently, in the next step, the odorous gas particles desorbed from the filter 125 of the gas trap 120 are supplied into the odor sensor chamber 200 having the odor sensor 210 installed therein (S104).

To this end, the first opening/closing valve 163 and the second opening/closing valve 164 may be controlled to be opened under the control of the controller. Thereafter, the first three-way valve 221 may be opened under the control of the controller 300 in a direction of enabling the connection pipe 150 and the odor sensor chamber 200 to communicate with each other. The second three-way valve 222 may be opened under the control of the controller 300 in a direction of enabling the odor sensor chamber 200 and the discharge pipe 202 to communicate with each other.

Furthermore, the suction pump 250 is driven under the control of the controller 300, and suction force resulting from the operation of the suction pump 250 is applied to the gas trap 120 in the prechamber 100 through the odor sensor chamber 200 and the connection pipe 150.

Therefore, the odorous gas particles desorbed from the rear portion of the filter 125 of the gas trap 120 may be supplied into the odor sensor chamber 200 without flowing to the bypass pipe 220 through the connection pipe 150.

Next, steps S105 to S108 are sequentially performed in the same manner as in the above-described embodiment.

In this manner, a low concentration odorous gas below a certain level is concentrated so as to make a high concentration odorous gas above a certain level, and then the high concentration odorous gas is supplied to an odor sensor so as to be sensed by the odor sensor. Thus, the low concentration odorous gas below the certain level may be easily measured.

For example, according to the present disclosure, it is possible to detect an odorous gas below a certain level that is emitted from an explosive or a drug. Accordingly, the odor measurement apparatus of the present disclosure may be usefully used in various industrial and inspection sites.

As is apparent from the above description, the present disclosure provides the following effects.

First, the present disclosure provides an odor measurement apparatus and an odor measurement method configured to measure an odorous gas having a low concentration below a certain level by concentrating the odorous gas having the low concentration below the certain level so as to make an odorous gas having a high concentration above a certain level, supplying the odorous gas having the high concentration to an odor sensor, and allowing the odor sensor to sense the odorous gas having the high concentration.

Second, the odor measurement apparatus according to the present disclosure is mounted on various vehicles, a future mobility system such as a purpose-built vehicle (PBV) and an urban air mobility (UAM) vehicle, and a robot, thereby enabling the odor measurement apparatus to reliably detect an odorous gas below a certain level that is emitted from an explosive or a drug. Accordingly, the odor measurement apparatus of the present disclosure may be usefully used in various industrial and inspection sites.

The present disclosure has been described in detail with reference to embodiments thereof. However, it should be appreciated by those having ordinary skill in the art that changes may be made in these embodiments without departing from the principles and spirit of the present disclosure, the scope of which is defined in the appended claims and equivalents thereto.

## Claims

1. An odor measurement apparatus, comprising:
a prechamber comprising:
an inlet disposed at a first side of the prechamber and configured to allow an odorous gas to be introduced into the prechamber; and
an outlet disposed at a second side of the prechamber and configured to allow the odorous gas to be discharged from the prechamber;
a flow pipe configured to connect the inlet to the outlet;
a gas trap mounted on the flow pipe and configured to collect odorous gas particles;
a first heater mounted on the prechamber and configured to heat the gas trap;
a first cooler mounted on the prechamber and configured to cool the gas trap;
an odor sensor chamber connected to the outlet of the prechamber through a connection pipe;
an odor sensor mounted in the odor sensor chamber and configured to sense the odorous gas desorbed from the gas trap; and
a controller configured to perform operating control of the first heater and the first cooler.

2. The odor measurement apparatus of claim 1, further comprising:
a bypass pipe comprising:
a first end connected to the connection pipe; and
a second end connected to a discharge pipe of the odor sensor chamber;
a first three-way valve mounted at a connection point between the connection pipe and the first end of the bypass pipe and controlled by the controller; and
a second three-way valve mounted at a connection point between the discharge pipe and the second end of the bypass pipe and controlled by the controller.

3. The odor measurement apparatus of claim 1 or 2, wherein the prechamber further comprises at least three inlets formed therein and configured to allow the odorous gas having a low concentration below a certain level to be smoothly introduced into the prechamber.

4. The odor measurement apparatus of anyone of claims 1-3, wherein a discharge pipe of the odor sensor chamber is connected to a suction pump driven under control of the controller.

5. The odor measurement apparatus of anyone of claims 1-4, wherein the odor sensor chamber comprises a second heater and a second cooler mounted on the odor sensor chamber, and
wherein the second heater is configured to heat the odor sensor under control of the controller, and the second cooler is configured to cool the odor sensor under the control of the controller.

6. The odor measurement apparatus of anyone of claims 1-5, wherein the gas trap comprises a case and a porous body, and
wherein the case is open at opposite sides of the case, and the porous body is installed in the case so as to adsorb the odorous gas particles.

7. The odor measurement apparatus of anyone of claims 1-6, wherein the gas trap comprises a case, multilayer metal plates, and a plurality of metal pins,
wherein the case is open at opposite sides of the case, the multilayer metal plates are stacked in the case, and the plurality of metal pins are mounted in a predetermined arrangement between the multilayer metal plates.

8. The odor measurement apparatus of anyone of claims 1-7, wherein the gas trap comprises a case and an electrostatic filter, and
wherein the case is open at opposite sides of the case, and the electrostatic filter is installed in the case so as to adsorb the odorous gas particles,
wherein the flow pipe comprises a guidance device mounted on the flow pipe and configured to cause the odorous gas to reversely flow from a rear portion of the gas trap to a front portion of the gas trap.

9. The odor measurement apparatus of claim 8, wherein the guidance device comprises a first guidance pipe and a second guidance pipe,
wherein the first guidance pipe comprises:
a first end connected to a predetermined position of the flow pipe connected to the front portion of the gas trap; and
a second end connected to a predetermined position of the flow pipe connected to the rear portion of the gas trap,
wherein the second guidance pipe comprises:
a first end connected to the flow pipe between the first end of the first guidance pipe and the front portion of the gas trap; and
a second end connected to the flow pipe between the second end of the first guidance pipe and the outlet of the prechamber,
wherein a first opening/closing valve is mounted on the flow pipe between the first end of the first guidance pipe and the first end of the second guidance pipe, and configured to be opened or closed under control of the controller, and
wherein a second opening/closing valve is mounted on the flow pipe between the second end of the first guidance pipe and the second end of the second guidance pipe, and configured to be opened or closed under control of the controller.

10. An odor measurement method, comprising:
allowing an odorous gas having a low concentration below a certain level to flow through a flow pipe connecting an inlet of a prechamber to an outlet of the prechamber;
collecting, by a gas trap mounted on the flow pipe, odorous gas particles for a predetermined time;
desorbing the odorous gas particles collected in the gas trap after a predetermined time;
supplying the odorous gas particles desorbed from the gas trap into an odor sensor chamber having an odor sensor installed therein; and
sensing, by the odor sensor, the odorous gas introduced into the odor sensor chamber.

11. The odor measurement method of claim 10, further comprising: driving a first cooler under control of a controller so as to allow the odorous gas particles to be adsorbed on the gas trap through cooling of the gas trap when collecting the odorous gas particles for the predetermined time.

12. The odor measurement method of claim 10 or 11, further comprising: driving a first heater under control of a controller so as to allow the odorous gas particles to be desorbed from the gas trap through heating of the gas trap when desorbing the odorous gas particles collected in the gas trap after the predetermined time.

13. The odor measurement method of anyone of claims 10-12, further comprising: driving, under control of a controller, a second cooler mounted on the odor sensor chamber so as to allow the odorous gas particles to be adsorbed on a surface of the odor sensor through cooling of the odor sensor when sensing the odorous gas introduced into the odor sensor chamber.

14. The odor measurement method of anyone of claims 10-13, further comprising: driving, under control of a controller, a second heater mounted on the odor sensor chamber so as to allow the odorous gas particles to be desorbed from a surface of the odor sensor through heating of the odor sensor after sensing the odorous gas introduced into the odor sensor chamber.

15. The odor measurement method of anyone of claims 10-14, wherein collecting, by the gas trap mounted on the flow pipe, the odorous gas particles for the predetermined time is repeatedly performed when a concentration of the odorous gas sensed by the odor sensor is below a certain level.
